# DEMANDE DE BREVET EUROPEEN

(11) **EP 0 974 839 A1**
(43) Date de publication de la demande: **26.01.2000**
(21) Numéro de dépôt: 99401609.5
(22) Date de dépôt: 25.06.1999
(51) Int. Cl.: G01N 33/24

(54) **Méthode de mesure rapide de l'indice de résistivité d'échantillons solides tels que des roches**

(30) Priorité: 24.07.1998 FR 9809542
(71) Demandeur: INSTITUT FRANCAIS DU PETROLE, 92852 Rueil-Malmaison Cedex (FR)
(72) Inventeur: Fleury, Marc, 78170 La Celle Saint Cloud (FR); Deflandre, François, 95120 Ermont (FR)

(57) **Abrégé**

- Méthode et dispositif de mesure de la courbe de l'indice de résistivité (Ir) d'un échantillon solide tel qu'un échantillon géologique indépendamment de la courbe de pression capillaire (Pc).
- On réalise des mesures de l'impédance complexe à plusieurs fréquences, de l'impédance d'un échantillon soumis à une pression radiale de confinement. au cours d'opérations de drainage ou d'imbibition d'un premier fluide initialement saturant, au travers d'une membrane semi-perméable, perméable à ce premier fluide. disposée à une première extrémité d'une cellule de confinement, ceci par injection d'un deuxième fluide sous pression à son extrémité opposée. On applique un ou plusieurs paliers de pression d'injection et, on mesure les variations continues de l'indice de résistivité (Ir) en fonction de la variation de saturation moyenne (Sw) sans attendre que s'établisse les équilibres capillaires. On utilise pour cela des électrodes plaquées contre l'échantillon à sa périphérie, ces électrodes ayant une extension longitudinale en rapport avec la longueur de l'échantillon et étant connectées à un impédancemètre. On applique un courant électrique au travers de l'échantillon et on détecte les variations de la différence de potentiel électrique qui en résultent. Les mesures de l'indice Ir peuvent être effectuées dans un temps relativement court : de quelques heures à quelques jours selon la perméabilité de la carotte.
- Applications à l'étude de zones souterraines par exemple.

## Description

L'invention a pour objet une méthode et un dispositif de mesure de la courbe de l'indice de résistivité d'un échantillon solide indépendamment de la courbe de pression capillaire.

La mesure de l'indice de résistivité de petites carottes est nécessaire pour obtenir une estimation précise de la saturation en eau à partir de données de diagraphie.

### ART ANTERIEUR

Différentes méthodes plus ou moins rapides et précises ont été proposées pour mesurer l'indice de résistivité de roches. Plusieurs combinaisons de ces techniques ont été proposées. Une méthode connue de mesure de l'indice de résistivité consiste essentiellement à combiner une technique de désaturation « à membrane semi-perméable » air-eau multi-carottes avec une technique de mesure de résistivité à deux électrodes et à calculer la saturation moyenne par différence de poids. Il s'avère que cette méthode est d'une grande imprécision et trop fortement dépendante de la qualité de la manipulation ; elle est très lente et ne prend pas en compte un éventuel effet de mouillabilité.

Différentes solutions utilisant le même principe ont été trouvées pour en améliorer la précision avec pour contrepartie une plus grande complexité de mise en oeuvre et un accroissement corrélatif des coûts. On peut par exemple :
- prendre des mesures séparément sur des carottes individuelles afin de mieux contrôler l'équilibre capillaire et ainsi obtenir des profils de saturation uniformes ;
- mettre en oeuvre une technique utilisant quatre électrodes afin d'éviter une surestimation de la résistance électrique ;
- mettre en oeuvre encore une technique d'injection continue afin d'accélérer les expériences lorsque seule la courbe Ir est nécessaire, comme décrit par exemple par de Waal et al., 1991 ;
- optimiser la durée du processus de désaturation grâce à l'utilisation d'une membrane microporeuse et en réduisant la longueur des carottes comme décrit par :
- Longeron D. et al.: « Water-Oil Capillarity Pressure and Wettability Measurements Using Micropore Membrane technique » SPE 30006, 1995 ; ou
- Fleury M. et al.: « Combined Resistivity and Capillarity Pressure Measurements using Micropore Membrane Technique » Proceeding of the International Symposium of the Society of Core Analysts, Montpellier 1996.

Cependant, lorsque la mesure de Ir est liée à la détermination des courbes de pression capillaire, il est très difficile de réduire la durée des expériences.

D'autres techniques de désaturation telles que la méthode centrifuge, peuvent être choisies et mises en oeuvre de manière similaire à la méthode « à membrane semi-perméable » à carottes multiples évoquée ci-dessus. La mise en oeuvre de la méthode centrifuge s'est également révélée imprécise du fait de l'accumulation de deux problèmes importants liés au profil de saturation et à la résistance de contact. Du fait de la relation connue d'Archie reliant Ir et Sw (Ir = Sw⁻ⁿ), les mesures sont très sensibles à la saturation. On peut alors déterminer également le profil de saturation (par exemple au cours d'une injection de fluide) et réduire la durée de l'expérience en utilisant un dispositif de mesure de la saturation locale in situ et des électrodes multiples comme décrit par :
- Jing et al. ; « resistivity Index from non Equilibrium Measurements using Detailed in situ Saturation Monitoring », SPE 26798 Offshore European Conference 1993.

La méthode selon l'invention permet de réaliser en continu une mesure de l'indice de résistivité d'un échantillon solide poreux, qui combine à la fois rapidité et précision et faible coût, en évitant les inconvénients des méthodes antérieures et notamment l'obligation d'avoir à surveiller la saturation in situ, qui s'avère longue et coûteuse.

### DEFINITION DE L'INVENTION

La méthode selon l'invention permet d'obtenir rapidement en continu les variations de l'indice de résistivité (Ir) d'un échantillon solide poreux, initialement saturé par un premier fluide, au moyen d'un dispositif comprenant une cellule de confinement allongée, des moyens pour exercer une pression radiale sur l'échantillon, des électrodes plaquées contre la paroi périphérique de l'échantillon, permettant l'application d'un courant électrique et la détection des différences de potentiel apparaissant entre des points distincts en réponse à l'application du courant électrique, les électrodes étant connectées à un appareil de mesure de l'impédance complexe de l'échantillon, un premier filtre semi-perméable, perméable au premier fluide et disposé sensiblement au contact d'une première extrémité de l'échantillon, et des moyens de pression pour l'injection sous pression d'un deuxième fluide au travers d'une deuxième extrémité de l'échantillon. La méthode est caractérisée en ce que :
- on utilise des électrodes dont l'extension longitudinale est relativement importante rapportée à la longueur de l'échantillon mais inférieure à cette longueur, choisies de façon à impliquer la plus grande partie possible du volume de l'échantillon dans les mesures d'impédance tout en évitant les court-circuits par les extrémités de l'échantillon susceptibles de fausser les mesures ; et
- on établit au moins un palier de pression d'injection du deuxième fluide et on réalise des mesures précises en continu des variations de l'impédance électrique complexe de l'échantillon à plusieurs fréquences durant une phase de déplacement du fluide saturant (phase de draînage ou phase d'imbibition), les mesures étant effectuées sans attendre que s'établisse un équilibre de la pression capillaire dans l'échantillon en réponse à chaque palier de pression.

On peut utiliser par exemple des électrodes dont la longueur est comprise entre ¼ et ¾ de la longueur de l'échantillon et de préférence est de l'ordre de la moitié de sa longueur.

Le dispositif selon l'invention comprend une cellule de confinement allongée, des moyens pour exercer une pression radiale sur l'échantillon, des électrodes plaquées contre la paroi périphérique de l'échantillon, permettant l'application d'un courant électrique et la détection des différences de potentiel apparaissant entre des points distincts en réponse à l'application du courant électrique, les électrodes étant connectées à un appareil de mesure de l'impédance de l'échantillon, un premier filtre semi-perméable, perméable au premier fluide et disposé sensiblement au contact d'une première extrémité de l'échantillon, et des moyens d'injection (14) pour l'injection sous pression d'un deuxième fluide au travers d'une deuxième extrémité de l'échantillon.

Le dispositif est caractérisé en ce que les électrodes ont une extension longitudinale relativement importante rapportée à la longueur de l'échantillon (entre ¼ et ¾ de la longueur de l'échantillon et de préférence de l'ordre de la moitié) mais inférieure à cette longueur, de façon à impliquer la plus grande partie possible du volume de l'échantillon dans les mesures d'impédance tout en évitant les court-circuits par les extrémités de l'échantillon.

La méthode selon l'invention, comparée aux méthodes antérieures, est particulièrement avantageuse en ce qu'elle permet :
- d'établir une courbe très précise d'indice de résistivité continue en drainage et en peu de temps (environ 2 jours pour un grès typique de 100 mD alors que la durée typique nécessaire en utilisant la technique d'injection continue est souvent de l'ordre d'une quinzaine de jours);
- la méthode n'est pas liée à un équilibre de la pression capillaire ;
- l'incidence de profils de saturation non uniformes au cours des mesures, s'avère négligeable. Ceci est dû à la combinaison de trois facteurs : (i) la technique de mesure de résistivité radiale, (ii) la présence de filtres semi-perméables côté sortie, (iii) la totalité du volume de la carotte est analysée au moyen de mesures électriques (ceci est vérifié lorsque le diamètre de la carotte est supérieur à sa longueur).

D'autres caractéristiques et avantages de la méthode selon l'invention, apparaîtront à la lecture de la description ci-après d'un exemple non limitatif de réalisation, en se référant aux dessins annexés où :

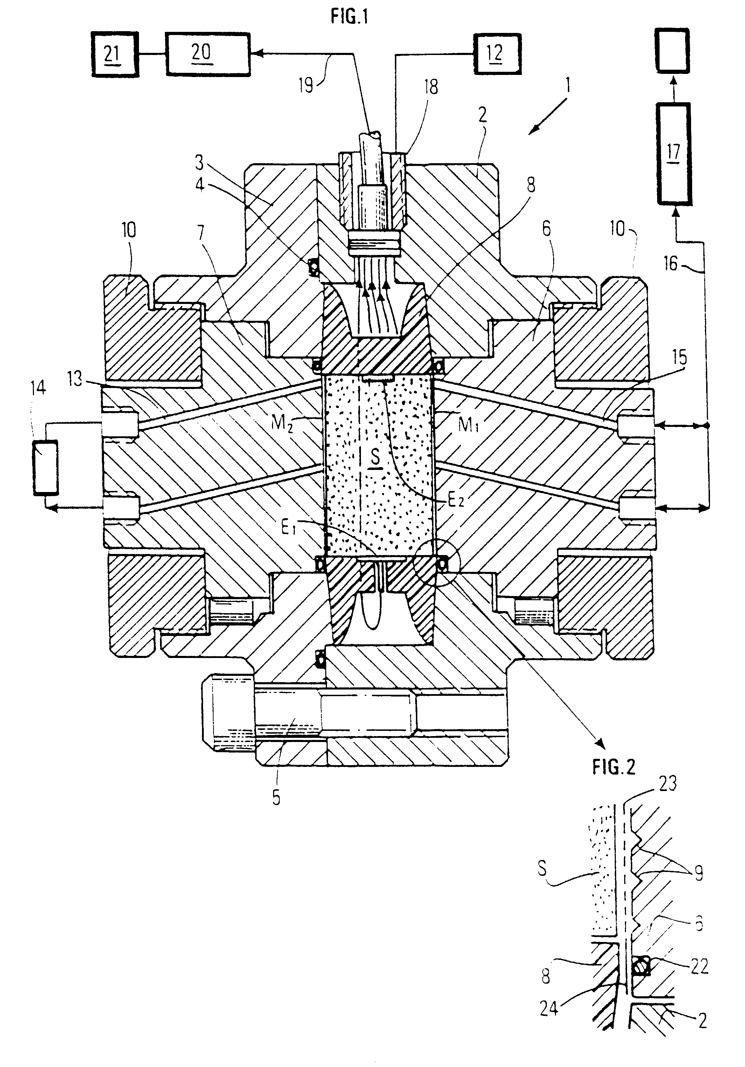

- la Fig.1 montre schématiquement en coupe longitudinale un dispositif de mise en oeuvre de la méthode ;
- la Fig.2 montre un détail de réalisation d'une membrane semi-perméable appliquée contre une face terminale de l'échantillon ;
- la Fig.3 montre l'agencement des électrodes permettant la mesure de l'indice de résistivité de la carotte ;
- la Fig.4a montre un exemple de courbe de variation au cours du temps, de la saturation en eau d'une carotte de perméabilité 80 mD au cours d'une expérience où trois niveaux de pression ont été successivement appliqués ;
- la Fig.4b montre la variation correspondante d'un indice n = -log(Ir)/log(Sw) ;
- la Fig.4c montre la variation correspondante de l'indice de résistivité en fonction de la variation de la saturation ;
- les Fig.5a, 5b, 5c montrent des figures respectivement analogues aux Fig.4a à 4c dans le cas d'un milieu poreux synthétique ayant une perméabilité de 2400 mD, obtenues au bout de 2 heures approximativement, avec application d'un seul seuil de pression de 45 kPa ;
- les Fig.6a, 6b, 6c montrent des courbes obtenues au cours de simulations numériques de déplacement de type « Porous Plate » : une courbe de production (Fig.6a) un profil de saturation (Fig.6b), et trois exposants de saturation nₚ, nₛ, n_{3D} obtenus en utilisant différents modèles (Fig.6c) ;
- la Fig.7 montre la courbe de pression capillaire utilisée dans ces simulations ; et
- les Fig.8a, 8b, 8c montrent des courbes analogues à celles des Fig.6a-6c dans le cas où l'on utilise un filtre céramique d'épaisseur 2 mm au lieu d'une membrane, avec un seul palier de pression à 50 kPa.

### DESCRIPTION DETAILLEE

La méthode est mise en en oeuvre en utilisant un système expérimental analogue à celui décrit dans le brevet EP 0.701.120 du demandeur.

Il comporte une cellule de confinement d'une carotte qui comporte un corps creux 1 constitué de deux manchons 2, 3 à symétrie cylindrique. Ils sont appliqués l'un contre l'autre par l'intermédiaire de joints d'étanchéité 4 et réunis par des vis 5. Les deux manchons comportent chacun une cavité axiale pour un embout 6, 7. L'échantillon S est placé à l'intérieur d'une pièce annulaire en élastomère en forme de U, constituant une gaîne 8. L'ensemble de l'échantillon S et de sa gaîne 8, est installé dans une cavité intérieure du manchon 3 et se trouve délimité axialement de part et d'autre par les deux embouts 6. 7. Du côté de l'embout 6, l'échantillon S est en contact avec un filtre semi-perméable M, mouillable par le fluide premier fluide (tel que de la saumure par exemple). Les deux embouts 6, 7 dont les faces en regard l'une de l'autre sont pourvues d'un réseau de rainures 9 (Fig.2), sont plaqués contre l'échantillon par vissage de deux écrous 10 dans les deux manchons 2, 3.

Des canaux 13 traversent l'embout 7 et font communiquer le réseau de rainures 11 sur sa face terminale, avec une première source 14 délivrant le deuxième fluide sous pression. De même, des canaux 15 traversent l'embout 6 et font communiquer le réseau de rainures 11 correspondant avec un circuit 16 de récupération du premier fluide draîné hors de l'échantillon du fait de l'injection du deuxième fluide. Un élément 17 est installé sur le circuit 16 pour mesurer le volume de fluide déplacé hors de l'échantillon S. On utilise de préférence un capteur capacitif de faible coût possédant une précision de 0,05 cc et d'une résolution de 0,01 cc, analogue à celui utilisé dans le dispositif décrit dans la demande de brevet FR 97/15.833 du demandeur.

Le dispositif comporte par exemple au moins un premier couple d'électrodes E1, E2 qui sont moulées à l'intérieur de la gaîne 8, de façon à s'appliquer étroitement contre la paroi périphérique de l'échantillon, permettant l'application d'un courant électrique. Au moyen de deux autres couples d'électrodes E'1, E'2, pareillement moulées, on mesure la différence de potentiel créée en réponse à l'application du courant électrique.

Cette affectation séparée des couples d'électrodes, l'un à l'application d'un courant, et l'autre, à la mesure de différences de potentiel, permet d'éviter les résistances dues aux contacts. Les électrodes sont par exemple de forme carrée et réalisées en Monel. L'extension angulaire d'une paire d'électrodes autour de l'échantillon est inférieure à 90°. Leur longueur doit être inférieure à la longueur de l'échantillon de façon à éviter les court-circuits électriques d'extrémité extérieurs à l'échantillon, directement au travers des fluides, ce qui fausseraient les mesures,. Cependant leur longueur doit être suffisamment importante rapportée à la longueur de l'échantillon de façon que les lignes de courant embrassent la plus grande partie de son volume avec une répartition relativement régulière. Cette longueur peut varier dans de notables proportions selon l'importance du diamètre de l'échantillon. Dans les expériences qui ont été réalisées, on a trouvé que la longueur des électrodes pouvait avantageusement être comprise entre ¼ et ¾ de la longueur de l'échantillon et de préférence être de l'ordre de la moitié de cette longueur.

Au travers d'un bouchon 18, l'espace à la périphérie de la gaîne 8 communique avec des moyens de pression 12 permettant l'injection d'un fluide sous pression qui exerce une pression radiale de confinement sur l'échantillon. La pression radiale de confinement autour de l'échantillon est par exemple de l'ordre de quelques Mpa, suffisante pour assurer un bon contact électrique des électrodes. Ainsi, dans des conditions normales, la résistance de contact est généralement du même ordre de grandeur que la résistance de l'échantillon qui doit être mesurée avec une faible saturation en eau.

Au travers d'un bouchon 18 qui est pourvu d'une traversée étanche, les fils conducteurs 19 reliés aux différentes électrodes E, E', sont connectés à un système de mesure de conductivité électrique 20, comportant un impédancemètre RLC à quatre électrodes interconnectées par couples, couplé avec un ensemble 21 d'acquisition de mesures.

Le système de mesure RLC 20 est adapté à mesurer les parties réelle et imaginaire de l'impédance complexe de la carotte. On vérifie à l'expérience que la partie imaginaire (liée aux effets capacitifs) est généralement négligeable par rapport à la partie réelle (résistance), sauf aux fréquences élevées.

Pour obtenir une parfaite étanchéité de l'enceinte de confinement de l'échantillon S, chaque embout 6, 7 comporte (Fig.2) une rainure dans sa paroi terminale pour un joint d'étanchéité 22, et on interpose entre le filtre M et l'embout 6 correspondant, une grille en métal 23 recouverte d'un enduit plastique. Cette grille est pourvue à sa périphérie d'une couronne non perforée 24 qui empêche toute fuite de fluide par contournement du filtre M.

L'ensemble est placé dans une enceinte thermostatée (non représentée).

Le filtre semi-perméable M (en céramique poreuse par exemple) est utilisé ici pour obtenir un profil de saturation particulier dans l'échantillon ou carotte, comme on le verra dans la suite de la description. Ils présentent également l'avantage de permettre une détermination facile de la saturation moyenne et de ne dépendre que faiblement des volumes morts des pièces d'extrémité. Le filtre M est ici mouillable à l'eau.

### FONCTIONNEMENT

L'échantillon S saturé avec le premier fluide est placé dans l'enceinte et l'on applique une pression de confinement radiale par l'ouverture 18.

On injecte alors par les canaux 13 un deuxième fluide tel que de l'huile à une première pression et l'on mesure en continu les variations de l'impédance complexe de l'échantillon pour plusieurs fréquences entre 0.1 et quelques 10 MHz. On peut prender par exemple les quatre fréquences suivantes : 0,1; 1; 10 et 100 kHz), qui sont enregistrées par l'ensemble d'acquisition 21. La résistance R à différentes saturations Sw est déduite de la partie réelle du signal à 1 kHz et l'indice de résistivité Ir = R/Ro où Ro est la résistance mesurée dans la cellule à une saturation de 100 %, Ro = R_{(Sw=1)}.

### Résultats expérimentaux

### Résultats

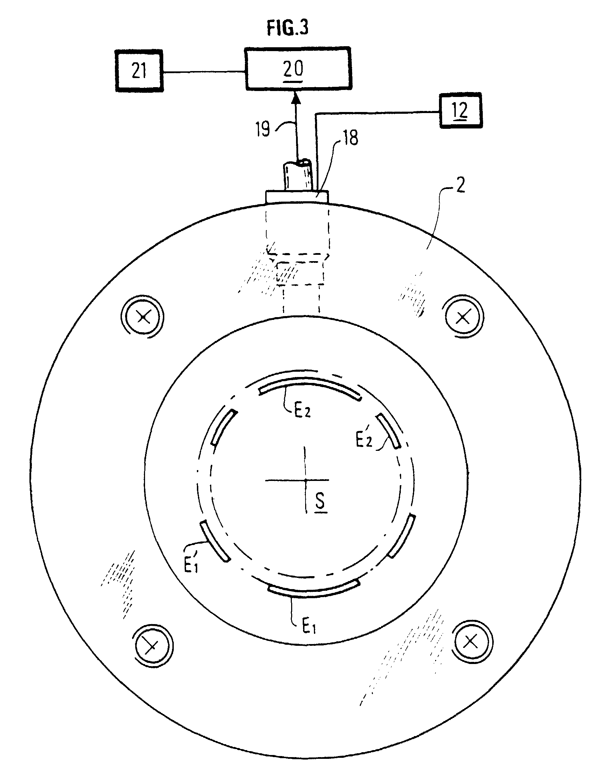

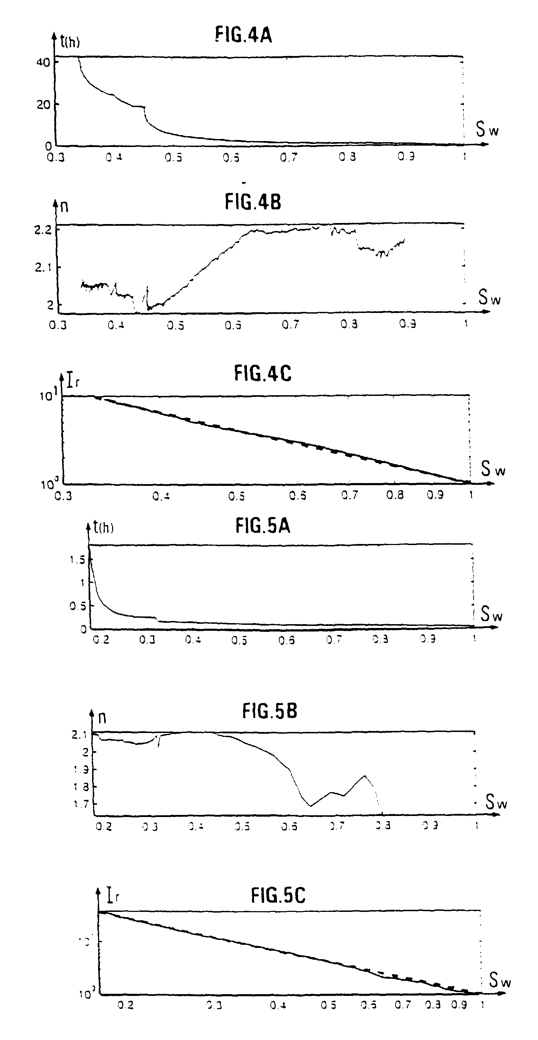

Deux expériences notamment ont été réalisées avec des membranes et avec des carottes de perméabilités très différentes (un grès des Vosges de 80 mD et une carotte synthétique de 2400 mD). Les fluides utilisés sont de la saumure (20 g/l NaCl) et du Soltrol 130. Pour la première carotte, trois niveaux de pression ont été successivement imposés (Ps = 10 kPa, 20 kPa et 500 kPa) mais l'équilibre n'a été obtenu qu'avec le premier niveau, comme le montre la courbe de production (Fig.4a). Pour ce type de carotte, la pression capillaire aux alentours du palier de la courbe se situe autour de 10 kPa. Lorsque l'indice de résistivité est porté en fonction de la saturation moyenne (Fig.4c), on observe que la courbe continue obtenue concorde bien avec une relation du type d'Archie sur l'ensemble de la gamme de saturation couverte. Aux alentours de Sw = 0,45, le fait que l'équilibre capillaire soit atteint, n'a pas d'effet sur la courbe Ir. L'écart local par rapport à une loi de puissance apparaît le plus nettement lorsque la pente locale n = -1n(Ir)/1n(<Sw>) est portée en fonction de la saturation (figure 3, courbe du milieu) : n est légèrement surestimé dans l'intervalle 0,6-0,9 où la production est très rapide.

Dans le second exemple (Fig.5a à 5c), la durée de l'expérience est beaucoup plus courte (2 heures) en raison de la perméabilité élevée de la carotte. D'autre part, on a appliqué ici un seul niveau de pression (Ps = 45 kPa); par rapport à l'exemple précédent, Ps est très supérieur à la pression aux alentours du palier de la courbe (environ 3 kPa). Là encore, la courbe Ir est bien décrite par une loi de puissance sur l'ensemble de la gamme de saturation depuis Sw = 1 jusqu'à 0,18 (figure 4, courbe inférieure). Le tracé de la pente locale (Fig.5b) indique une sous-estimation de n dans la zone de forte saturation en eau (Sw = 0,6 à 1).

Les résultats expérimentaux indiquent que les mesures d'indice de résistivité peuvent être effectuées en un temps très court, qui peut varier entre quelques heures et quelques dizaines d'heures en fonction de la perméabilité de la carotte, sans qu'il soit nécessaire d'atteindre un équilibre capillaire. Les courbes Ir sont déterminées avec une grande précision et sont étonnamment insensibles aux profils de saturation sur la majeure partie de la gamme de saturation. Un niveau de pression unique élevé donne des écarts plus importants que ceux relevés avec 2 ou 3 niveaux de pression dans le cas d'une forte saturation en eau. mais fournit néanmoins une bonne précision pour une faible saturation en eau.

### Interprétation

On a procédé à des simulations numériques afin de comprendre la surprenante insensibilité des mesures de résistivité aux profils de saturation non uniformes. Deux types de simulations ont été réalisés :
- des simulations numériques unidimensionnelles de processus de déplacement du type « à paroi semi-perméable » en vue de calculer le profil de saturation pour un ensemble donné de courbes de pression capillaire et de perméabilité relative. Elles ont permis de démontrer que les courbes de perméabilité relative obtenues dans ce type de déplacement, sont déterminées de manière médiocre ;

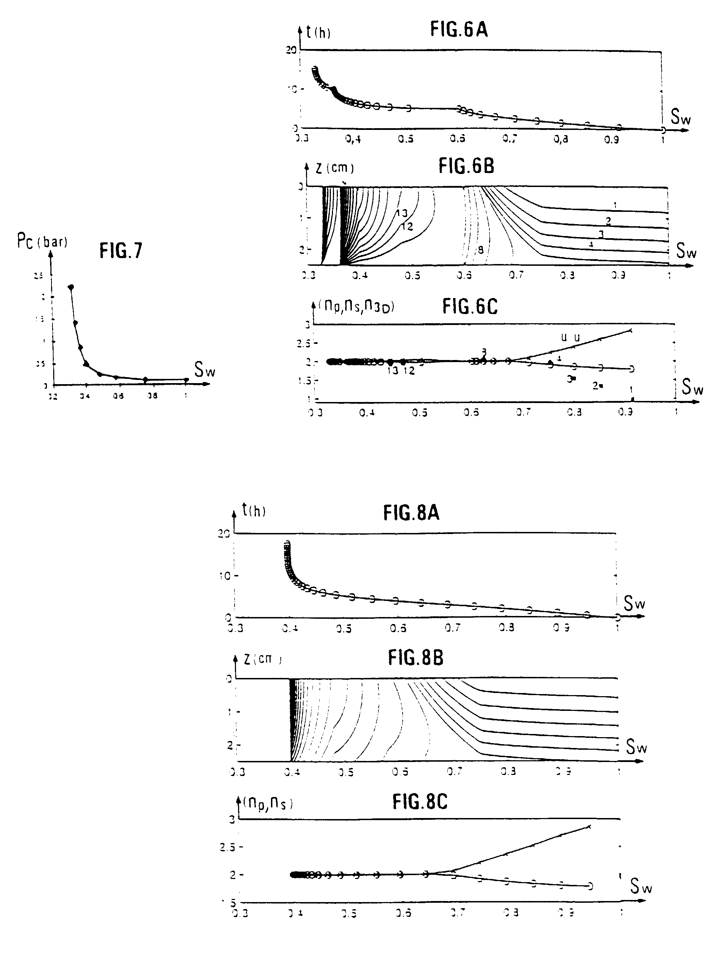

Des simulations tridimensionnelles du champ électrique ont été faites afin de reproduire la variation de la résistance entre différents profils. Pour celles présentées sur les Fig.6a-6c, les conditions aux limites où les paramètres suivants ont été imposés :
- perméabilité de la carotte de 100 mD ;
- pression capillaire obtenue à partir de mesures prises sur des carottes similaires (Fig.7) ;
- un ensemble arbitraire de courbes de perméabilité relative de type Corey, les exposants relatifs à l'huile et à l'eau étant tous deux égaux à 3, et Kro(Swirr) = 0,8 ;
- on a utilisé une membrane M1 mouillable à l'eau située à z = 2,5 cm (z étant le sens d'allongement de la carotte), d'une épaisseur de 0,1 mm et d'une perméabilité de 0,1 mD (valeurs typiques obtenues à partir d'essais) ;
- l'huile est injectée à z = 0 avec trois pressions différentes (16,5, 100 et 200 kPa) ;
- exposant de saturation n = 2.

Pour un profil de saturation donné (numéroté de 1 à 13), on calcule trois exposants de saturation n = -1n(R/Ro)/1n(<Sw(z)>);
- np (modèle en parallèle) : chaque point du profil de saturation Sw(z) est une résistance d'une valeur R(z) = Sw(z)⁻ⁿ; la résistance moyenne en parallèle est R = Rp = 1/<1/R(z)> = <Sw(z)ⁿ>; ce modèle devrait décrire les mesures utilisant des électrodes radiales ;
- ns (modèle en série) : la résistance moyenne en série est R = Rs = <R(z)> = 1/<Sw(z)⁻ⁿ>; ce modèle devrait décrire les mesures de face à face ;
- n_{3D} (modèle de réservoir analogique 3D) : l'indice de résistivité Ir = Qo/Q, où Qo est le débit de référence calculé pour une saturation uniforme Sw = 1. Q est calculé pour un profil de perméabilité K(z) = Sw(z)ⁿ.

Lors de l'analyse des profils de saturation (Fig.6a-6c), il convient de distinguer deux domaines :
- un domaine à saturation en eau élevée (approximativement, dans le cas présent. Sw = 0,7 à 1) où les simulations indiquent que l'huile n'a pas atteint la membrane ;
- un domaine à saturation en eau moyenne et faible (Sw < 0,7) où la saturation en huile peut être plus importante côté sortie (côté membrane, z = 2,5 cm) que côté entrée z = 0 (profil de saturation 12 par exemple). Cet effet est dû à la légère chute de pression de la membrane qui permet une désaturation rapide près de la sortie et dépend également du choix des courbes Kr.

Ces simulations ont permis de conclure que :
- en comparant ns, np et n3D (Fig.6a-6c), une configuration de mesure de résistivité sensible au modèle parallèle est nettement moins influencée par les profils de saturation non uniformes. Ceci est notamment le cas du fait de la géométrie radiale des électrodes. Les calculs en 3D indiquent la même tendance : n_{3D} est très proche de la valeur d'entrée n = 2 pour Sw < 0,8 ;
- il est important de mesurer la résistance de l'ensemble de la carotte ; en effet, pour un grand nombre de profils, ns et np sont proches parce que 1/<Sw(z)⁻ⁿ> ou <Sw(z)ⁿ> ne sont pas très différents de Sw(z)ⁿ ;
- pour une saturation en eau élevée (Sw > 0,8), les simulations indiquent une sensibilité beaucoup plus importante aux profils de saturation : n3D passe de 2 à 1 (Fig.6), une valeur éloignée du modèle parallèle et en contradiction avec les mesures. Deux explications peuvent être retenues : (i) le champ électrique est fortement modifié par le profil de saturation et le modèle parallèle ne peut pas être appliqué; (ii) les simulations ne reproduisent pas le processus de percolation qui apparaît avec une saturation Sw élevée et des caractéristiques de digitation sont plus représentatives de la répartition tridimensionnelle de l'huile dans le milieu poreux; les profils de saturation calculés ne sont donc pas pertinents.

## Revendications

1. Méthode pour obtenir rapidement en continu les variations de l'indice de résistivité (Ir) d'un échantillon solide poreux, initialement saturé par un premier fluide, au moyen d'un dispositif comprenant une cellule de confinement allongée (1), des moyens (12) pour exercer une pression radiale sur l'échantillon, des électrodes (E1, E2) plaquées contre la paroi périphérique de l'échantillon, permettant l'application d'un courant électrique et la détection des différences de potentiel apparaissant entre des points distincts en réponse à l'application du courant électrique, les électrodes étant connectées à un appareil (20) de mesure de l'impédance de l'échantillon, un premier filtre (M) semi-perméable, perméable au premier fluide et disposé sensiblement au contact d'une première extrémité de l'échantillon, et des moyens de (14) pour l'injection sous pression d'un deuxième fluide au travers d'une deuxième extrémité de l'échantillon, caractérisée en ce que :
- on utilise des électrodes dont l'extension longitudinale est relativement importante rapportée à la longueur de l'échantillon mais inférieure à cette longueur, choisies de façon à impliquer la plus grande partie possible du volume de l'échantillon dans les mesures d'impédance tout en évitant les court-circuits par les extrémités de l'échantillon. ; et
- on établit au moins un palier de pression d'injection du deuxième fluide et on réalise des mesures précises en continu des variations de l'impédance électrique complexe de l'échantillon à plusieurs fréquences durant une phase de déplacement du fluide saturant, les mesures étant effectuées sans attendre que s'établisse un équilibre de la pression capillaire dans l'échantillon en réponse à chaque palier de pression.

2. Méthode selon la revendication 1, caractérisée en ce que l'on utilise des électrodes dont la longueur est comprise entre ¼ et ¾ de la longueur de l'échantillon.

3. Méthode selon la revendication 1, caractérisée en ce que l'on utilise des électrodes dont la longueur est de l'ordre de la moitié de la longueur de l'échantillon.

4. Méthode selon l'une des revendications précédentes, caractérisée en ce que l'on réalise des mesures précises en continu des variations de l'impédance électrique complexe de l'échantillon à plusieurs fréquences durant une phase de draînage.

5. Méthode selon l'une des revendications précédentes, caractérisée en ce que l'on réalise des mesures précises en continu des variations de l'impédance électrique complexe de l'échantillon à plusieurs fréquences durant une phase d'imbibition.

6. Dispositif pour obtenir rapidement en continu les variations de l'indice de résistivité (Ir) d'un échantillon solide poreux, initialement saturé par un premier fluide, comprenant une cellule de confinement allongée (1), des moyens (12) pour exercer une pression radiale sur l'échantillon, des électrodes (E1, E2) plaquées contre la paroi périphérique de l'échantillon, permettant l'application d'un courant électrique et la détection des différences de potentiel apparaissant entre des points distincts en réponse à l'application du courant électrique, les électrodes étant connectées à un appareil (20) de mesure de l'impédance de l'échantillon, un premier filtre (M) semi-perméable, perméable au premier fluide et disposé sensiblement au contact d'une première extrémité de l'échantillon, et des moyens d'injection (14) pour l'injection sous pression d'un deuxième fluide au travers d'une deuxième extrémité de l'échantillon, caractérisé en ce que les électrodes ont une extension longitudinale relativement importante rapportée à la longueur de l'échantillon mais inférieure à cette longueur, de façon à impliquer la plus grande partie possible du volume de l'échantillon dans les mesures d'impédance tout en évitant les court-circuits par les extrémités de l'échantillon.

7. Dispositif selon la revendication 6, caractérisé en ce que la longueur des électrodes est comprise entre ¼ et ¾ de la longueur de l'échantillon et de préférence être de l'ordre de la moitié de cette longueur.

8. Dispositif selon la revendication 6, caractérisé en ce que la longueur des électrodes est de l'ordre de la moitié de la longueur de l'échantillon.
